(19) **Europäisches Patentamt / European Patent Office / Office européen des brevets**

(11) **EP 3 484 846 B1**

(12) **EUROPÄISCHE PATENTSCHRIFT**

(45) Veröffentlichungstag und Bekanntmachung des Hinweises auf die Patenterteilung:
**23.09.2020 Patentblatt 2020/39**

(51) Int Cl.:
**C07C 51/23** [(2006.01)] **C07C 53/02** [(2006.01)]

(21) Anmeldenummer: **17740383.9**

(86) Internationale Anmeldenummer:
**PCT/EP2017/068054**

(22) Anmeldetag: **17.07.2017**

(87) Internationale Veröffentlichungsnummer:
**WO 2018/015351 (25.01.2018 Gazette 2018/04)**

(54) **VERFAHREN ZUR KATALYTISCHEN ERZEUGUNG VON AMEISENSÄURE UND REGENERATION DES DABEI EINGESETZTEN KATALYSATORS BEI GERINGEM ÜBERDRUCK**

METHOD FOR CATALYTICALLY PRODUCING FORMIC ACID AND REGENERATING THE CATALYST USED IN THE PROCESS WITH LITTLE OVERPRESSURE

PROCÉDÉ DE PRODUCTION CATALYTIQUE D'ACIDE FORMIQUE ET RÉGÉNÉRATION DU CATALYSEUR UTILISÉ À CET EFFET À PRESSION POSITIVE RÉDUITE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2016 DE 102016213099**

(43) Veröffentlichungstag der Anmeldung:
**22.05.2019 Patentblatt 2019/21**

(73) Patentinhaber: **OxFA GmbH**
**96110 Scheßlitz (DE)**

(72) Erfinder:
• **JBACH, Hermann Wolf**
**96120 Bischberg (DE)**

• **KOHLER, Florian**
**90419 Nürnberg (DE)**
• **SCHMIDT, Matthias**
**91362 Pretzfeld (DE)**
• **SCHOLZ, Gunthard**
**96163 Gundelsheim (DE)**

(74) Vertreter: **Dr. Gassner & Partner mbB**
**Wetterkreuz 3**
**91058 Erlangen (DE)**

(56) Entgegenhaltungen:
**DE-A1-102011 077 232**

**Beschreibung**

Die Erfindung betrifft ein Verfahren zur katalytischen Erzeugung von Ameisensäure und Regeneration des dabei eingesetzten Katalysators. Ein derartiges Verfahren ist aus der EP 2 473 467 B1 bekannt. Dabei wird ein als Katalysator dienendes Polyoxometallat-Ion der allgemeinen Formel $[PMo_xV_yO_{40}]^{5-}$ bei einer Temperatur unterhalb von 120 °C mit einem alpha-Hydroxyaldehyd, einer alpha-Hydroxycarbonsäure, einem Kohlenhydrat oder einem Glycosid in einer flüssigen Lösung in Kontakt gebracht, wobei 6 < x < 11 und 1 < y < 6 und x + y = 12, wobei x und y jeweils eine ganze Zahl ist. Der dabei reduzierte Katalysator wird durch Oxidation wieder in seinen Ausgangszustand versetzt.

Gemäß der DE 10 2011 077 232 A1 und der dazu korrespondierenden EP 2 473 467 B1 ist es vorteilhaft, wenn das Inkontaktbringen unter einem Sauerstoffpartialdruck von 1 bis 500 bar, insbesondere 50 bis 75 bar, erfolgt. Je höher der Sauerstoffpartialdruck ist, desto schneller erfolgt die Oxidation des bei dem Verfahren reduzierten Katalysators. Ein Reaktionsgefäß zur effektiven Durchführung des aus der EP 2 473 467 B1 bekannten Verfahrens muss einem erheblichen Druck standhalten können. Damit ein solches Reaktionsgefäß sicher ist, muss es verhältnismäßig dickwandig ausgeführt sein und Materialverbindungen innerhalb des Reaktionsgefäßes müssen hohen Sicherheitsanforderungen genügen. Auch die Gewährleistung der Sicherheit bei der Durchführung des Verfahrens ist wegen des für eine effektive Oxidation des Katalysators erforderlichen verhältnismäßig hohen Drucks bei der Reaktion aufwändig. Die Durchführung des aus der EP 2 473 467 B1 bekannten Verfahrens ist deshalb verhältnismäßig teuer.

Aus der DE 10 2014 212 995 A1 ist ein Verfahren zur katalytischen Erzeugung von Carbonsäuren bekannt, bei dem ein Polyoxometallat der Form $H_{3+y}[PMo_xV_yO_{40}]$ als Katalysator dient und mit einem kohlenstoffhaltigen Festbrennstoff in einer flüssigen Lösung in Kontakt gebracht wird, wobei 6 < y < 13, 1 < x < 6 und x + y = 12 und wobei x und y jeweils eine ganze Zahl ist. Der Katalysator kann dabei mit einem sauerstoffhaltigen Gasgemisch mit einem Sauerstoffpartialdruck zwischen 0,1 bar und 100 bar regeneriert werden. Die Temperatur bei dem Verfahren kann unterhalb von 150 °C liegen. Mit dem Verfahren kann Essigsäure oder Ameisensäure erzeugt werden. Das während der Ameisensäureherstellung entstehende Abgas wird aus dem Prozess herausgeführt.

Aufgabe der vorliegenden Erfindung ist es, ein kostengünstig und effizient durchzuführendes Verfahren zur katalytischen Erzeugung von Ameisensäure und Regeneration des dabei eingesetzten Katalysators anzugeben.

Die Aufgabe wird durch die Merkmale des Patentanspruchs 1 gelöst. Zweckmäßige Ausgestaltungen ergeben sich aus den Merkmalen der Patentansprüche 2 bis 18.

Erfindungsgemäß ist ein Verfahren zur katalytischen Erzeugung von Ameisensäure und Regeneration des dabei eingesetzten Katalysators vorgesehen, wobei ein als Katalysator dienendes Vanadyl-Ion, Vanadat-Ion oder Polyoxometallat-Ion der allgemeinen Formel $[PMo_xV_yO_{40}]^{n-}$ bei einer Temperatur oberhalb von 70 °C, insbesondere oberhalb von 80 °C, insbesondere oberhalb von 90 °C, und unterhalb von 160 °C, insbesondere unterhalb von 150 °C, insbesondere unterhalb von 140 °C, insbesondere unterhalb von 130 °C, insbesondere unterhalb von 120 °C, mit einem Substrat in Form eines alpha-Hydroxyaldehyds, einer alpha-Hydroxycarbonsäure, eines Kohlenhydrats, eines Glycosids oder eines eine Kohlenstoffkette enthaltenden Polymers mit mindestens einer wiederholt als Substituent an die Kohlenstoffkette gebundenen OH-Gruppe und/oder mit einem wiederholt in der Kohlenstoffkette enthaltenen O-, N-oder S-Atom in einer flüssigen Lösung in einem Gefäß in Kontakt gebracht wird. Dabei ist 6 ≤ x ≤ 11 und 1 ≤ y ≤ 6 und 3 < n < 10 und x + y = 12, wobei n, x und y jeweils eine ganze Zahl ist. Der dabei reduzierte Katalysator wird durch Oxidation wieder in seinen Ausgangszustand versetzt. Dazu wird die Lösung mit einem einen Volumenanteil von mindestens 18 %, insbesondere mindestens 19 %, insbesondere mindestens 20 %, insbesondere mindestens 30 %, insbesondere mindestens 40 %, insbesondere mindestens 50 %, insbesondere mindestens 70 %, insbesondere mindestens 90 %, insbesondere mindestens 95 %, Sauerstoff enthaltenden Gas bei einem Druck von mindestens 2 bar, insbesondere mindestens 3 bar, insbesondere mindestens 4 bar, insbesondere mindestens 5 bar, insbesondere mindestens 6 bar, insbesondere mindestens 7 bar, insbesondere mindestens 8 bar, insbesondere mindestens 9 bar, insbesondere mindestens 10 bar, insbesondere mindestens 11 bar, insbesondere mindestens 12 bar, insbesondere mindestens 13 bar, und höchstens 16 bar, insbesondere höchstens 15 bar, insbesondere höchstens 12 bar, insbesondere höchstens 10 bar, insbesondere höchstens 9 bar, insbesondere höchstens 8 bar, insbesondere höchstens 7 bar, insbesondere höchstens 6 bar, mittels einer Mischvorrichtung oder über eine flüssigkeitsundurchlässige gasdurchlässige Membran in Kontakt gebracht. Der Volumenanteil des Gases, der nicht Sauerstoff ist, kann Stickstoff enthalten oder aus Stickstoff bestehen.

Das Vanadyl-Ion, d. h. $VO^{2+}$, kann beispielsweise in Form von $VOSO_4$ oder $VOPO_4$ als Katalysator in die Lösung eingebracht werden. Das Vanadat-Ion, d. h. ein Oxoanion von Vanadium in der Oxidationsstufe +5, kann beispielsweise als $VO_4^{3-}$, z. B. in einer Lösung von Natriumorthovanadat ($Na_3VO_4$), oder als das polymere $[VO_3]_n^{n-}$, beispielsweise in Form von $NaVO_3$ (Natriummetavanadat), als Katalysator in die Lösung eingebracht werden.

Bei der Reaktion entstehendes CO und/oder $CO_2$ tritt aus der Lösung in das Gas über und wird in einer solchen Menge abgeführt, dass ein Volumenanteil von CO und $CO_2$ zusammen an dem Gas 80 % nicht übersteigt. Dies kann z. B. dadurch erreicht werden, dass das die Lösung kontaktierende Gas oder zumindest ein Teil dieses Gases permanent oder stoßweise spätestens bei Erreichen des CO-und/oder $CO_2$-Volumenanteils von 80 % durch frisches Gas ersetzt wird, d. h., dass der die Lösung kontaktierende Gasraum mit die Lösung bis dahin nicht kontaktierendem Gas gespült

EP 3 484 846 B1

wird. Das stoßweise Ersetzen durch frisches Gas kann so erfolgen, dass intermittierend ein Teil des die Lösung kontaktierenden Gases oder das gesamte die Lösung kontaktierende Gas durch frisches Gas ersetzt wird. Das Volumen bzw. das pro Zeiteinheit zugeführte Volumen des zugeführten Gases beim permanenten oder intermittierend stoßweisen Ersetzen des die Lösung kontaktierenden Gases kann in Abhängigkeit des gemessenen Volumenanteils von CO und $CO_2$ zusammen an dem Gas, insbesondere an dem den Gasraum verlassenden Gas, reguliert werden. Alternativ kann das Abführen von entstehendem CO und/oder $CO_2$ dadurch erreicht werden, dass das CO und/oder das $CO_2$, spätestens bei Erreichen dieses Volumenanteils von 80 %, aus dem Gas abgesondert wird. Unter dem "Volumenanteil von CO und $CO_2$ zusammen" wird die Summe der Volumenanteile von CO und $CO_2$ verstanden. Unter einem Gas wird hier ein Gas oder ein Gasgemisch verstanden. Die Zusammensetzung des Gases ändert sich im Laufe des Verfahrens ständig z. B. durch das Entstehen von CO und/oder $CO_2$, durch das Abführen von entstandenem CO und/oder $CO_2$ und durch den Entzug von Sauerstoff durch die Oxidation des Katalysators. Bei dem anfänglich eigesetzten Gas und beim frischen Gas zum Spülen des die Lösung kontaktierende Gasraums kann es sich beispielsweise um Luft handeln, welche üblicherweise einen Sauerstoffgehalt von etwa 20,9 bis 21 % hat. Für das Verfahren benötigter Sauerstoff kann auch durch eine Elektrolyse von Wasser gewonnen werden. Der dabei gleichzeitig entstehende Wasserstoff kann durch Verbrennen zum Erwärmen der Lösung oder in einem anderen optionalen Verfahrensschritt, z. B. zur Rektifikation entstandener Ameisensäure, verwendet werden. Der Wasserstoff kann auch zur chemischen Hydrierung von bei dem Verfahren entstehendem $CO_2$ zu Ameisensäure verwendet werden.

Das erfindungsgemäße Verfahren kann im Hinblick auf die Lösung und/oder das umzusetzende Edukt, d. h. das alpha-Hydroxyaldehyd, die alpha-Hydroxycarbonsäure, das Kohlenhydrat, das Glycosid oder das Polymer, als Batch-Verfahren, als Fed-Batch-Verfahren oder als kontinuierliches Verfahren in dem Gefäß oder in mehreren Gefäßen durchgeführt werden. Bei dem Batch-Verfahren werden der Lösung nach Beginn des Verfahrens keine weitere Lösung und kein weiteres Edukt, d. h. kein weiteres alpha-Hydroxyaldehyd, keine weitere alpha-Hydroxycarbonsäure, kein weiteres Kohlenhydrat, kein weiteres Glycosid und kein weiteres Polymer, zugesetzt. Beim Fed-Batch-Verfahren handelt es sich um ein Verfahren, bei dem das Gefäß anfänglich nur mit einem Teil der Lösung oder einem Teil des bei dem Verfahren umzusetzenden Edukts gefüllt ist und bei dem im Laufe des Verfahrens in einem oder mehreren Schritten weitere Lösung oder weiteres Edukt zugesetzt wird. Bei dem kontinuierlichen Verfahren wird/werden der anfänglich in dem Gefäß enthaltenen Lösung und dem anfänglich in dem Gefäß enthaltenen Edukt permanent Lösung und/oder unverbrauchtes Edukt zugeführt und Lösung mit erzeugter Ameisensäure und optional verbrauchtes Edukt entzogen. Sowohl beim Batch-Verfahren als auch beim Fed-Batch-Verfahren als auch beim kontinuierlichen Verfahren kann das Zuführen und das Abführen des Gases unabhängig von dem Zuführen und dem Entziehen der Lösung und/oder des Edukts, beispielsweise wie oben beschrieben, erfolgen.

Bei dem Verfahren kann das CO und/oder das $CO_2$ von dem Gas abgesondert werden. Das Absondern kann beispielsweise durch eine Verflüssigung von $CO_2$ durch Komprimieren oder mittels einer für $CO_2$ und/oder CO durchlässigen, nicht oder nur eingeschränkt jedoch für Sauerstoff durchlässigen Membran oder einer Kombination von zwei oder mehreren Membranen unterschiedlicher Durchlässigkeit, z. B. einer ersten für $CO_2$ und CO und nicht oder nur eingeschränkt für $O_2$ durchlässigen Membran und einer zweiten für $CO_2$ und nicht oder nur eingeschränkt für CO durchlässigen Membran, erfolgen. Durch die Kombination von Membranen unterschiedlicher Durchlässigkeit kann das abgesonderte Gas separiert bzw. gesondert abgeschieden und dann einer weiteren Verwendung zugeführt werden. Alternativ kann/können das CO und/oder $CO_2$ auch mittels Druckwechsel-Adsorption von dem Gas abgesondert werden. Durch das Absondern kann ein relativ reines $CO_2$ gewonnen und als Produkt verwertet werden. Das abgesonderte $CO_2$ kann auch mittels elektrochemischer Reduktion in Ameisensäure überführt werden.

Bei einer Ausgestaltung des Verfahrens beträgt der Volumenanteil von CO und $CO_2$ zusammen an dem Gas mindestens 20 %, insbesondere mindestens 25 %, insbesondere mindestens 30 % und/oder das bei der Reaktion entstehende und in das Gas übergehende CO und/oder $CO_2$ wird in einer solchen Menge abgeführt, dass der Volumenanteil von CO und $CO_2$ zusammen an dem Gas 70 %, 60 %, 55 %, 50 %, 45 %, 40 %, 35 %, 30 %, 25 % oder 20 % nicht übersteigt. Das bei der Reaktion entstehende und in das Gas übergehende CO und/oder $CO_2$ kann dadurch in einer solchen Menge abgeführt werden, dass der Volumenanteil von CO und $CO_2$ zusammen an dem Gas 80 %, 70 %, 60 %, 55 %, 50 %, 45 %, 40 %, 35 %, 30 %, 25 % oder 20 % nicht übersteigt, dass das die Lösung kontaktierende Gas oder zumindest ein Teil dieses Gases permanent oder stoßweise spätestens bei Erreichen dieses Volumenanteils von 80 %, 70 %, 60 %, 55 %, 50 %, 45 %, 40 %, 35 %, 30 %, 25 % oder 20 % durch frisches Gas ersetzt wird oder indem das CO und/oder das $CO_2$, spätestens bei Erreichen dieses Volumenanteils von 80 %, 70 %, 60 %, 55 %, 50 %, 45 %, 40 %, 35 %, 30 %, 25 % oder 20 %, von dem Gas abgesondert wird. Frisches Gas ist Gas, welches die Lösung bis dahin nicht kontaktiert hat, einen Sauerstoffgehalt von mindestens 18 % aufweist und bei dem der Volumenanteil von CO und $CO_2$ zusammen geringer ist als bei dem ersetzten Gas. Bei einem Volumenanteil von CO und $CO_2$ zusammen von höchstens 80 % kann der minimale Volumenanteil von Sauerstoff 20 % betragen, wenn sonst kein weiteres Gas enthalten ist. Entsprechend kann der minimale Volumenanteil von Sauerstoff bei einem Volumenanteil von CO und $CO_2$ zusammen von höchstens 70 %, 60 %, 55 %, 50 %, 45 %, 40 %, 35 %, 30 %, 25 % oder 20 % jeweils 30 %, 40 %, 45 %, 50 %, 55 %, 60 %, 65 %, 70 %, 75 % oder 80 % betragen, wenn sonst kein weiteres Gas enthalten ist. Der genannte

3

Volumenanteil von CO und $CO_2$ zusammen an dem Gas von mindestens 20 %, insbesondere mindestens 25 %, insbesondere mindestens 30 % hat sich wirtschaftlich als günstig erwiesen, wenn der sonstige Volumenanteil zu einem großen Teil aus Sauerstoff besteht, weil der angereicherte Sauerstoff verhältnismäßig teuer ist und, insbesondere beim Absondern von CO und $CO_2$ über eine Membran, stets das Risiko eines Verlusts eines Teils des Sauerstoffs besteht.

Der bei der Reaktion zur Bildung der Ameisensäure reduzierte Katalysator wird durch Oxidation wieder in seinen Ausgangszustand versetzt. Im Sinne der Erfindung wird unter einem Katalysator also auch ein Stoff verstanden, der während des Verfahrens durch Reduktion verändert und durch Oxidation wieder in seinen Ausgangszustand versetzt wird.

Die flüssige Lösung kann eine wässrige Lösung sein. Unter der Lösung wird eine Lösung des Katalysators verstanden. Das Substrat und andere Bestandteile können darin gelöst oder auch nur suspendiert vorliegen. Die Lösung kann ein Lösungsmittel zum Lösen des Substrats umfassen. Das Vorsehen eines solchen Lösungsmittels ist nicht erforderlich, wenn das alpha-Hydroxyaldehyd, die alpha-Hydroxycarbonsäure, das Kohlenhydrat, das Glycosid oder das Polymer bereits in flüssiger Form vorliegt. Dann kann die flüssige Lösung auch eine Lösung des Katalysators in dem Substrat sein. Unter einem alpha-Hydroxyaldehyd wird jedes Molekül verstanden, bei welchem eine OH-Gruppe direkt an einem C-Atom gebunden ist, wobei an dem C-Atom auch direkt das C-Atom einer Aldehyd-Gruppe gebunden ist. Unter einer alpha-Hydroxycarbonsäure wird jedes Molekül verstanden, bei welchem eine OH-Gruppe direkt an einem C-Atom gebunden ist, wobei an dem C-Atom auch direkt das C-Atom einer Carboxy-Gruppe gebunden ist. Unter einem alpha-Hydroxyaldehyd und unter einer alpha-Hydroxycarbonsäure kann auch jede Substanz verstanden werden, welche ein alpha-Hydroxyaldehyd oder eine alpha-Hydroxycarbonsäure enthält.

Die Erfinder der vorliegenden Patentanmeldung haben festgestellt, dass das bei der katalytischen Umsetzung der genannten Substrate bei einem limitierten Druck entstehende CO und/oder $CO_2$ einen unerwartet stark limitierenden Einfluss auf die Ausbeute an Ameisensäure und/oder die Geschwindigkeit der Erzeugung von Ameisensäure hat. Sie haben weiterhin festgestellt, dass durch ein Geringhalten des Volumenanteils von CO und $CO_2$ zusammen an dem Gas, so dass der Volumenanteil 80 %, 70 %, 60 %, 55 %, 50 %, 45 %, 40 %, 35 %, 30 %, 25 % oder 20 % nicht übersteigt, einerseits die Ausbeute und/oder die Geschwindigkeit der Bildung der Ameisensäure gesteigert werden kann und andererseits das Verfahren mit einem verhältnismäßig geringen Druck von maximal 16 bar, sogar maximal 15 bar, sogar maximal 12 bar, sogar maximal 10 bar, sogar maximal 9 bar, sogar maximal 8 bar, sogar maximal 7 bar, sogar maximal 6 bar, mit ausreichender Ausbeute und/oder Geschwindigkeit der Bildung der Ameisensäure bzw. ausreichender spezifischen Produktleistung (Raum-Zeit-Ausbeute) durchgeführt werden kann. Das Verfahren ermöglicht es sogar, die Oxidation des Katalysators mit Luft beim oben genannten Druck ausreichend effizient durchzuführen. Da die Vorrichtung zur Durchführung des Verfahrens dadurch weniger Druck standhalten muss, kann die Vorrichtung deutlich kostengünstiger bereitgestellt werden als eine Vorrichtung zur Durchführung des aus der EP 2 473 467 B1 bekannten Verfahrens zur katalytischen Erzeugung von Ameisensäure.

Bei einer Ausgestaltung des Verfahrens beträgt der Volumenanteil des Sauerstoffs am die Lösung kontaktierenden Gas mindestens 45 %, insbesondere mindestens 50 %, insbesondere mindestens 60 %, insbesondere mindestens 70 %, insbesondere mindestens 80 %, insbesondere mindestens 85 %, insbesondere mindestens 90 %, insbesondere mindestens 95 %, insbesondere mindestens 96 %, insbesondere mindestens 98 %, insbesondere 100 %. Der Gasdruck kann mindestens 6 bar, insbesondere mindestens 7 bar, insbesondere mindestens 8 bar, insbesondere mindestens 9 bar, insbesondere mindestens 10 bar, insbesondere mindestens 11 bar, insbesondere mindestens 12 bar, insbesondere mindestens 13 bar, betragen. Bei einer Ausgestaltung des Verfahrens beträgt der Gasdruck des Gases höchstens 15 bar, insbesondere höchstens 12 bar, insbesondere höchstens 10 bar, insbesondere höchstens 9 bar, insbesondere höchstens 8 bar, insbesondere höchstens 7 bar, insbesondere höchstens 6 bar. Das erfindungsgemäße Verfahren kann auch so durchgeführt werden, dass der Partialdruck von CO und $CO_2$ zusammen an einer Grenzfläche der Lösung zum Gas 13 bar, insbesondere 12 bar, insbesondere 10 bar, insbesondere 5 bar, insbesondere 2 bar, insbesondere 1 bar, nicht übersteigt. Unter dem "Partialdruck von CO und $CO_2$ zusammen" wird die Summe der Partialdrücke von CO und $CO_2$ verstanden. Durch eine Verringerung des Partialdrucks von CO und/oder $CO_2$ bzw. CO und $CO_2$ zusammen lässt sich die Effizienz des Verfahrens steigern.

Das bei der Reaktion entstehende und in das Gas übergehende CO und/oder $CO_2$ kann in einer solchen Menge abgeführt werden, dass bei auf einen Höchstwert beschränktem Druck oder bei konstant gehaltenem Druck ein Sauerstoffpartialdruck in dem Gas durch das CO und/oder $CO_2$ um nicht mehr als 10 %, insbesondere nicht mehr als 8 %, insbesondere nicht mehr als 6 %, insbesondere nicht mehr als 4 %, insbesondere nicht mehr als 2 %, insbesondere nicht mehr als 1 %, vermindert wird. In Abhängigkeit von dem Substrat, insbesondere dem Verhältnis von Kohlenstoff zu Wasserstoff in dem Substrat und der Konzentration des Substrats, kann bei der Erzeugung der Ameisensäure mehr oder weniger CO und/oder $CO_2$ entstehen. Üblicherweise entsteht stets mehr $CO_2$ als CO, wobei das Verhältnis von entstehendem $CO_2$ zu entstehendem CO ebenfalls vom Substrat aber auch von den Prozessbedingungen abhängt. Üblicherweise liegt das Verhältnis von entstehendem $CO_2$ : entstehendem CO zwischen 3 : 1 und 20 : 1. Dadurch, dass der Sauerstoffpartialdruck konstant oder zumindest verhältnismäßig konstant gehalten wird, indem die Menge des, beispielsweise durch Spülen mit frischem Gas, abzuführenden COs und/oder $CO_2$s in Abhängigkeit vom Sauerstoffpar-

tialdruck geregelt wird, kann der Sauerstoffpartialdruck in einem für den Prozess günstigen Bereich gehalten werden. Durch die genannte Regelung kann das Verfahren an unterschiedliche Substrate und Substratkonzentration angepasst werden. Dadurch kann die katalytische Ameisensäureerzeugung stets mit größtmöglicher Effektivität oder zumindest mit verhältnismäßig hoher Effektivität erfolgen.

Zur Oxidation des Katalysators kann ein Teil der flüssigen Lösung aus dem Gefäß ausgeleitet, mit dem Gas in Kontakt gebracht und anschließend wieder dem Rest der flüssigen Lösung zugeführt werden. Der Teil der flüssigen Lösung kann mit Überdruck dem Rest der flüssigen Lösung zugeführt werden. Dabei kann darin gelöster Sauerstoff auch zu einer Oxidation eines Teils des im Rest der flüssigen Lösung enthaltenen Katalysators beitragen.

Das Zuführen des gegenüber dem Rest der flüssigen Lösung unter einem höheren Druck stehenden Teils der flüssigen Lösung unter diesem Druck zum Rest der flüssigen Lösung kann wegen der Druckdifferenz zwischen dem Teil und dem Rest der flüssigen Lösung auch als ein Einströmen des Teils in den Rest der flüssigen Lösung gestaltet und zu einer Durchmischung der flüssigen Lösung und zu einer Dispersion des Gases in der flüssigen Lösung genutzt werden. Das Einströmen kann durch mindestens eine entsprechend gestaltete, insbesondere in der Strömungsrichtung und/oder in ihrem Auslassdurchmesser variierbare, Düse erfolgen. Durch Veränderung des Auslassdurchmessers kann die Ausströmgeschwindigkeit des Teils der flüssigen Lösung aus der Düse verändert werden.

Um das CO und/oder $CO_2$ bei Übersteigen des genannten Volumenanteils abzuführen, kann das Gefäß eine durch ein Ventil regulierte nach außen führende Öffnung aufweisen. Um zu verhindern, dass entstandene Ameisensäure dampfförmig verloren geht, kann am Gefäß oder außerhalb des Gefäßes eine Kondensationsvorrichtung bereitgestellt werden, an der entstandener Ameisensäuredampf, insbesondere vor dem Ersetzen des die Lösung kontaktierenden Gases durch frisches Gas, ggf. zusammen mit Wasser, kondensieren kann, so dass ein dadurch entstandenes Kondensat in das Gefäß zurückgeleitet, zur Absonderung der Ameisensäure von dem darin enthaltenen Wasser einer Extraktion zugeführt oder zur Absonderung der Ameisensäure abgeleitet werden kann. Vorteilhaft ist dabei, dass der Katalysator bei der Temperatur des Verfahrens nicht verdampft, so dass die Ameisensäure so gewonnen werden kann, ohne dass eine Absonderung der Ameisensäure vom Katalysator erforderlich ist. Alternativ kann in dem Gas entstandene dampfförmige Ameisensäure, insbesondere vor dem Ersetzen des die Lösung kontaktierenden Gases durch frisches Gas, mittels eines zur Absorption von Ameisensäure geeigneten Absorptionsmittels, insbesondere einem linearen Alkohol, insbesondere 1-Pentanol, 1-Hexanol, 1-Heptanol, 1-Oktanol oder 1-Dekanol, oder einem Amid, insbesondere einem N,N-Dialkylcarbonsäureamid, insbesondere Dipentylformamid, N-(n-Hexadecyl)formamid, N,N-Di-n-butylformamid (DBFA), N-Di-n-acetamid, N-Methyl-N-hetpylformamid, N-n-butyl-N-2-ethylhexyl-formamid oder N-n-butyl-N-cyclohexylformamid, aus dem Gas oder dem oben genannten Kondensat absorbiert und anschließend davon desorbiert werden. Das Desorbieren kann beispielsweise durch eine Entspannungsverdampfung (Flash-Verdampfung), d. h. ein Verdampfen durch Absenken des Drucks, bewirkt werden.

Das Absorptionsmittel kann danach durch einen Wärmetauscher wieder auf eine gewünschte für die Absorption geeignete Temperatur gebracht und erneut zur Absorption eingesetzt werden.

Es ist auch möglich, dass die Ameisensäure aus dem Gas von einer Base, insbesondere einer wässrigen Lösung von NaOH oder KOH, absorbiert wird und die daraus resultierende Salzlösung zur weiteren Verwendung abgeleitet wird.

Der Teil der flüssigen Lösung kann kontinuierlich oder in, insbesondere regelmäßigen, Intervallen aus dem Gefäß ausgeleitet, mit dem Gas bei einem Druck von mindestens 2 bar, 3 bar, 4 bar oder 5 bar und höchstens 16 bar beaufschlagt und anschließend wieder dem Rest der flüssigen Lösung kontinuierlich oder in, insbesondere regelmäßigen, Intervallen zugeführt werden.

Auch das Oxidieren kann kontinuierlich oder in, insbesondere regelmäßigen, Intervallen durchgeführt werden. Dies ermöglicht es, den in dem Gefäß enthaltenen Katalysator während des gesamten Verfahrens in ausreichender Menge in oxidierter und damit zur Umsetzung des alpha-Hydroxyaldehyds, der alpha-Hydroxycarbonsäure, des Kohlenhydrats, des Glycosids oder des Polymers geeigneter Form bereitzustellen, ohne dass dazu eine allzu große Menge des verhältnismäßig teuren Katalysators eingesetzt werden muss.

Bei der Ausgestaltung des Verfahrens, bei der zur Oxidation des Katalysators ein Teil der flüssigen Lösung aus dem Gefäß ausgeleitet wird, dann mit dem Gas in Kontakt gebracht und anschließend wieder dem Rest der flüssigen Lösung zugeführt wird, kann der Teil der flüssigen Lösung z. B. durch einen Wärmetauscher, bei konstanter oder zumindest nahezu konstanter Temperatur gehalten werden.

Um zu verhindern, dass grobe Feststoffe in den in Strömungsrichtung nachgelagerten Teil der Vorrichtung zur Durchführung des Verfahrens gelangen und dort beispielsweise Ventile oder sonstige Teile der Vorrichtung zusetzen oder sonst in ihrer Funktion beeinträchtigen, kann beim Ausleiten des Teils der flüssigen Lösung aus dem Gefäß ein Hydrozyklon, ein Dekanter oder ein Filter, insbesondere ein Gegenstromfilter, ein Mikrofilter oder ein Ultrafilter, vorgesehen sein.

Bei einer Ausgestaltung des erfindungsgemäßen Verfahrens liegt das alpha-Hydroxyaldehyd, die alpha-Hydroxycarbonsäure, das Kohlenhydrat, das Glycosid oder das Polymer in der flüssigen Lösung in Form von darin verteilten Feststoffen vor. Die Feststoffe können fein verteilt oder grob verteilt in der flüssigen Lösung vorliegen.

Bei dem Polymer kann es sich um ein Polyester, ein Polyamin oder ein Polyamid, insbesondere Polyhexamethylenadipinsäureamid (Nylon), handeln. Das Polymer kann ein Polymer ohne Weichmacher sein. Weichmacher können die Aktivität des Katalysators negativ beeinflussen.

Alpha-Hydroxyaldehyde, alpha-Hydroxycarbonsäuren, Kohlenhydrate und Glycoside kommen in einer großen Zahl nachwachsender Rohstoffe, wie beispielsweise Stärke, Zellulose oder Hemizellulose vor. Stärke, Zellulose und Hemizellulose fallen in großen Mengen als Produkt aus Ackerpflanzen oder beim industriellen Holzaufschluss, beispielsweise zur Papierherstellung an.

Bei dem alpha-Hydroxyaldehyd, der alpha-Hydroxycarbonsäure, dem Kohlenhydrat oder dem Glycosid kann es sich um ein Monosaccharid, insbesondere einer Aldose, Disaccharid, Oligosaccharid oder Polysaccharid, Stärke, Zellulose, Hemizellulose, Glucose, Saccharose, Xylose, Zellobiose, Xylan, ein Hetereooligosaccharid, ein Heteropolysaccharid, Glycolsäure oder Milchsäure oder einen das alpha-Hydroxyaldehyd, die alpha-Hydroxycarbonsäure, das Kohlenhydrat oder das Glycosid enthaltenden Reststoff oder, insbesondere nachwachsenden, insbesondere unbehandelten, Rohstoff handeln. Unbehandelt bedeutet dabei, dass der Rohstoff zuvor nicht chemisch aufgeschlossen worden ist. Bei dem Reststoff oder dem nachwachsenden Rohstoff kann es sich um eine Pflanze, einen Pilz oder Bakterien oder Bestandteile von Pflanzen, Pilzen oder Bakterien, Holz, insbesondere in Form von Holzmehl oder Holzspänen, Papier, insbesondere Altpapier, Algen, Cyanobakterien, Silage oder eine proteinreiche Substanz, insbesondere Getreide-schlempe, Trester, oder Biertreber, handeln. Das alpha-Hydroxyaldehyd, die alpha-Hydroxycarbonsäure, das Kohlenhydrat oder das Glycosid kann auch ein Gemisch aus mindestens zwei der genannten Substanzen umfassen oder aus mindestens einer der genannten Substanzen oder dem Gemisch entstanden sein, wie dies z. B. bei Braunkohle oder Torf der Fall ist.

Viele der genannten Rohstoffe fallen als Nebenprodukte, beispielsweise bei der Papierherstellung oder Holzverarbeitung an. Sie stehen damit als günstiger Ausgangsstoff für das erfindungsgemäße Verfahren zur Verfügung. Das erfindungsgemäße Verfahren kann dadurch sehr kostengünstig durchgeführt werden. Dazu trägt auch die Oxidation des bei der katalysierten Reaktion reduzierten Katalysators (= Reoxidation) bei, die eine sehr lange Verwendung des Katalysators erlaubt.

Die Mischvorrichtung kann einen statischen Mischer, eine Reaktionsmischpumpe, eine Düse, insbesondere eine Venturi-Düse oder eine Sprühdüse, und/oder einen Gaseintragsrührer umfassen. Die Mischvorrichtung kann dazu aus mindestens einer der genannten Mischeinrichtungen bestehen oder eine Mehrzahl davon oder auch eine Mehrzahl verschiedener der genannten Mischeinrichtungen umfassen. Die Mischvorrichtung kann so ausgelegt sein und so betrieben werden oder die Membran kann so aufgebaut sein, dass dadurch die Oberfläche der Lösung, d. h. die Grenzfläche zwischen der Lösung und dem Gas, mindestens um den Faktor 1000, insbesondere mindestens um den Faktor 5000, insbesondere mindestens um den Faktor 10000, vergrößert wird. Die Größe dieses Faktors hängt je nach Mischvorrichtung häufig nicht nur von der Mischvorrichtung selbst, sondern auch der Art des Betriebs, etwa der Rührgeschwindigkeit bei einem Gaseintragsrührer, der Drehzahl bei einer Reaktionsmischpumpe oder der Strömungsgeschwindigkeit bei einem statischen Mischer, einer Venturi-Düse oder einer Sprühdüse ab.

Das Absondern von CO und/oder $CO_2$ ermöglicht es, dass das Gas, insbesondere ohne einen Druckabfall von mehr als 2,5 bar, insbesondere mehr als 2 bar, insbesondere mehr als 1,5 bar, insbesondere mehr als 1 bar, insbesondere mehr als 0,5 bar, mittels einer Fördervorrichtung, wie einem Kompressor, einer Venturi-Düse oder einem Gebläse, in einem Kreislauf geführt wird. Dadurch kann auch ein höherer Sauerstoffgehalt als in Luft in dem Gas aufrechterhalten werden, ohne dass Sauerstoff oder zumindest eine wesentliche Menge von Sauerstoff außer durch Oxidation des Katalysators aus dem Kreislauf entweicht. Ein verhältnismäßig hoher Sauerstoffgehalt des Gases erhöht gegenüber einem Gas mit niedrigerem Sauerstoffgehalt, wie z. B. Luft, die Effizienz der Oxidation des Katalysators.

Bei einer Ausgestaltung wird das gesamte Verfahren als kontinuierlicher Prozess durchgeführt. Dazu kann beispielsweise kontinuierlich oder in, insbesondere regelmäßigen, Intervallen eine Teilmenge der flüssigen Lösung aus dem Gefäß ausgeleitet, darin enthaltene Ameisensäure, beispielsweise in Form eines Formiats, abgesondert und die verbleibende Teilmenge der flüssigen Lösung wieder dem Rest der flüssigen Lösung zugeführt werden. Ein dazu geeignetes Verfahren ist beispielsweise in der WO 2016/078698 A1 beschrieben.

Aus der Lösung oder einer danach der Lösung wieder zugeführten Teilmenge der Lösung können der Katalysator und die Ameisensäure mittels mindestens eines Extraktionsmittels, insbesondere unter Aufrechterhaltung eines pH-Werts von höchstens 3, insbesondere höchstens 2,5, insbesondere höchstens 2, in der Lösung, abgesondert werden. Eine Aufrechterhaltung des pH-Werts von höchstens 3, insbesondere höchstens 2,5, insbesondere höchstens 2, kann erreicht werden, indem nur ein Teil der Ameisensäure aus der Lösung oder der Teilmenge der Lösung extrahiert wird und so viel Ameisensäure in der Lösung belassen wird, dass dadurch der pH-Wert nicht über 3, insbesondere nicht über 2,5, insbesondere nicht über 2, steigt. Das Extraktionsmittel für den Katalysator und die Ameisensäure kann identisch sein. Es kann sich dabei insbesondere um ein die Ameisensäure und den Katalysator extrahierendes polares organisches Extraktionsmittel handeln, welches beim Mischen mit der flüssigen Lösung eine Phasengrenze zwischen der Lösung und dem Extraktionsmittel bildet. Das Extraktionsmittel kann ein Extraktionsmittel sein, welches für eine Extraktion des in einer Konzentration von 1,5 Gew.-% in Wasser enthaltenen Katalysators bei 40 °C einen Verteilungskoeffizienten für den Katalysator aufweist, welcher mindestens um den Faktor 7, insbesondere 8, insbesondere 9, insbesondere 10, insbesondere 15, insbesondere 20, insbesondere 25, insbesondere 30, größer ist als ein Verteilungskoeffizient für eine Extraktion der in einer Konzentration von 5 Gew.-% in Wasser enthaltenen Ameisensäure bei 40 °C. Der Verteilungskoeffizient K ist wie folgt definiert:

$$K = \frac{\text{(Konzentration Ameisensäure bzw. Katalysator) im Extraktionsmittel}}{\text{(Konzentration Ameisensäure bzw. Katalysator) im Wasser}}$$

Das Extraktionsmittel kann dadurch den Katalysator am Beginn der Extraktion schneller extrahieren als die Ameisensäure. "Schneller" bedeutet dabei insbesondere, dass pro Zeiteinheit ein größerer Gewichtsprozentanteil vom Katalysator an dem insgesamt in der Lösung enthaltenen Katalysator als von der Ameisensäure an der insgesamt in der Lösung enthaltenen Ameisensäure in das Extraktionsmittel übergeht.

Zur Zurückhaltung von in der Flüssigkeit verteilten Feststoffen in dem Gefäß kann beim Ausleiten der Teilmenge der flüssigen Lösung aus dem Gefäß ein Hydrozyklon, ein Dekanter oder ein Filter, z. B. ein Gegenstromfilter, ein Mikrofilter oder ein Ultrafilter, vorgesehen sein.

Das Extraktionsmittel kann vor der Extraktion mit dem Katalysator gesättigt werden oder der abgesonderte Katalysator kann aus dem Extraktionsmittel abgesondert und der flüssigen Lösung in dem Gefäß zugeführt werden. Dadurch wird vermieden, dass die Konzentration des Katalysators in dem Gefäß soweit absinkt, dass dadurch keine effiziente Erzeugung der Ameisensäure mehr möglich ist.

Das Absondern kann in einem zwei-stufigen Prozess erfolgen, indem die Lösung in einem ersten Schritt der Extraktion mit einer ersten Menge des Extraktionsmittels für eine erste Zeit zur Extraktion des Katalysators und in einem zweiten Schritt der Extraktion mit einer zweiten Menge des Extraktionsmittels für eine zweite Zeit zur Extraktion der Ameisensäure extrahiert wird. Der in dem ersten Schritt der Extraktion extrahierte Katalysator kann wieder der flüssigen Lösung in dem Gefäß zugeführt werden. Das Extraktionsmittel kann beispielsweise ein Amid, insbesondere ein N,N-Dialkylcarbonsäureamid, insbesondere Dipentylformamid, N-(n-Hexadecyl)formamid, N,N-Di-n-butylformamid (DBFA), N-Di-n-acetamid, N-methyl-N-heptylformamid, N-n-butyl-N-2-ethylhexylformamid oder N-n-butyl-N-cyclohexylformamid, sein. Bei der Extraktion einer wässrige Ameisensäure und den Katalysator enthaltenden Lösung wird bei einer kurzen Extraktion mit verhältnismäßig wenig eines solchen Extraktionsmittels zunächst hauptsächlich der Katalysator und nur wenig Ameisensäure extrahiert. Bei einer anschließenden Extraktion mit demselben Extraktionsmittel wird dann hauptsächlich Ameisensäure aus der Lösung extrahiert. Um zu vermeiden, dass bei der ersten Extraktionsstufe Ameisensäure aus der Lösung extrahiert wird, kann das Extraktionsmittel vorher mit Ameisensäure gesättigt werden. Dem Extraktionsmittel kann ein, insbesondere unpolares, Additiv zugesetzt werden. Bei dem unpolaren Additiv kann es sich z. B. um Petroleum, eine Fraktion von Petroleum, n-Hexan, n-Oktan, n-Decan, Oleylalkohol, Toluol, Dibutylether oder Tri-n-butylphosphat handeln.

Das Absondern des Katalysators kann mittels Fällung als Salz, insbesondere gleichzeitig mit einer Fällung von extrahierter Ameisensäue als Formiat, oder mittels einer weiteren Extraktion mit einem polaren weiteren Extraktionsmittel, insbesondere einem Lösungsmittel der Lösung, und einem Temperaturwechsel des Extraktionsmittels und/oder einer Erhöhung des pH-Werts des Extraktionsmittels, insbesondere durch Zusatz eines Carbonats, beispielsweise Natriumcarbonat, erfolgen. Der pH-Wert kann alternativ oder zusätzlich auch durch Zusatz eines Hydroxids, beispielsweise KOH oder NaOH, erhöht werden. Bei dem weiteren Extraktionsmittel kann es sich beispielsweise um Wasser oder die Lösung, in welcher die Ameisensäure erzeugt wird, handeln. Die Extraktion durch das weitere Extraktionsmittel und den Temperaturwechsel und/oder die Erhöhung des pH-Werts können durch das unpolare Additiv begünstigt werden. Bei der Fällung des Katalysators können auch gleichzeitig Formiate mitgefällt werden. In einem solchen Fall wäre eine nachfolgende Absonderung des Katalysators von den Formiaten, beispielsweise durch einen weiteren Extraktionsschritt, erforderlich. Der ausgefällte Katalysator kann, bevor er der Lösung wieder zugeführt wird, in einer, insbesondere mit Ameisensäure, angesäuerten wässrigen Lösung aufgelöst werden. Durch das Ansäuern kann der pH-Wert an den pH-Wert der Lösung angepasst werden. Dadurch kann verhindert werden, dass sich der pH-Wert der Lösung durch das Zugeben des gelösten Katalysators ändert.

Die extrahierte Ameisensäure kann mittels Fällung als Formiat oder mittels Destillation vom Extraktionsmittel abgesondert werden. Bei der Destillation kann es sich um eine sogenannte Flash-Destillation handeln. Eine solche Destillation ist nur einstufig und daher sehr unkompliziert.

Nachfolgend wird die Erfindung anhand von Ausführungsbeispielen näher erläutert. Es zeigen:

Fig. 1    eine schematische Darstellung einer Vorrichtung zur Durchführung des erfindungsgemäßen Verfahrens,

Fig. 2    eine schematische Darstellung einer alternativen Vorrichtung zur Durchführung des erfindungsgemäßen Verfahrens mit einem statischen Mischer,

Fig. 3 eine schematische Darstellung einer alternativen Vorrichtung zur Durchführung des erfindungsgemäßen Verfahrens mit einer Venturi-Düse und

Fig. 4 eine graphische Darstellung der Ausbeute an Ameisensäure in Abhängigkeit von dem Volumenanteil von CO und $CO_2$ zusammen an dem Gas.

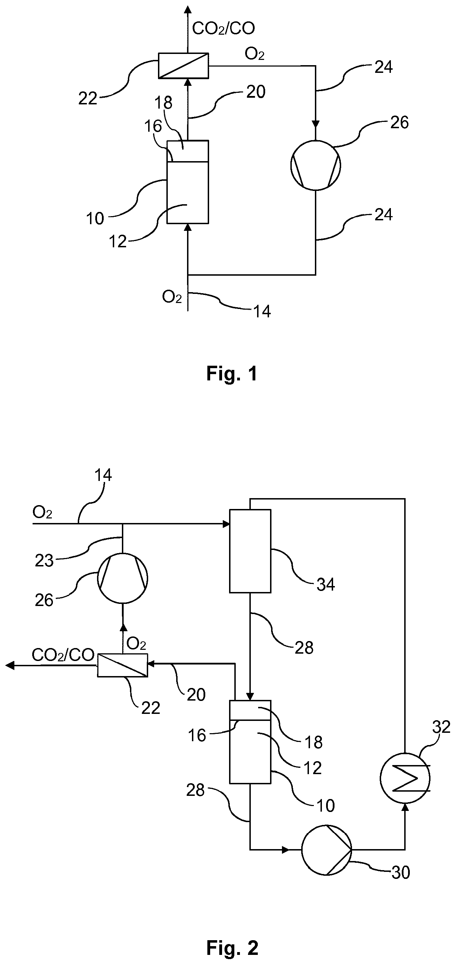

Fig. 1 zeigt ein Gefäß 10, welches die Lösung 12 mit dem Katalysator und einem Substrat in Form eines alpha-Hydroxyaldehyds, einer alpha-Hydroxycarbonsäure, eines Kohlenhydrats, eines Glycosids oder eines eine Kohlenstoffkette enthaltenden Polymers mit mindestens einer wiederholt als Substituent an die Kohlenstoffkette gebundenen OH-Gruppe und/oder mit einem wiederholt in der Kohlenstoffkette enthaltenen O-, N- oder S-Atom enthält.

In das Gefäß 10 mündet die Zuführleitung 14 zum Zuführen des einen Volumenanteil von mindestens 18 % Sauerstoff enthaltenden Gases 18. Das Zuführen des Gases 18 kann von außen oder über die unten beschriebene Rückführleitung 24 erfolgen. Die Oberfläche der Lösung 12 bildet die Grenzfläche 16 zum Gas 18, welches sich im Gefäß 10 oberhalb der Lösung 12 sammelt und einen Gasdruck von mindestens 5 bar und höchstens 33 bar aufweist. Das Gas wird über die Abführleitung 20 dem Separator 22 zugeführt. In dem Separator 22 erfolgt eine Absonderung von CO und/oder $CO_2$ vom Gas mittels einer Membran, welche für CO und $CO_2$, nicht aber oder nur eingeschränkt für $O_2$ durchlässig ist, oder mittels Druckwechsel-Adsorption. Das verbleibende Gas wird über die Rückführleitung 24 und den Kompressor 26 wieder in die Zuführleitung 14 eingespeist, um wieder in die Lösung 12 eingeblasen zu werden. Der nicht durch Oxidation des Katalysators verbrauchte Sauerstoff im Gas wird so im Kreis geführt, während das CO und/oder das $CO_2$, welches bei der Reaktion in der Lösung entsteht, abgesondert wird.

Fig. 2 zeigt eine alternative Vorrichtung, welche die Durchführung des erfindungsgemäßen Verfahrens mit höherer Effizienz erlaubt als die in Fig. 1 dargestellte Vorrichtung. Die in dem Gefäß 10 enthaltene Lösung 12 des oben genannten Substrats wird durch die Flüssigkeitsleitung 28 aus dem Gefäß 10 ausgeleitet und mittels der Pumpe 30 zunächst durch den Wärmetauscher 32 und dann durch den statischen Mischer 34 gepumpt. Dem statischen Mischer 34 wird über die Zuführleitung 14 ein einen Volumenanteil von mindestens 18 % Sauerstoff enthaltendes Gas mit einem Gasdruck von mindestens 5 bar und höchstens 33 bar von außen oder aus der unten beschriebenen Einspeiseleitung 23 zugeführt und in dem statischen Mischer 34 unter diesem Druck mit der Lösung 12 in Kontakt gebracht. Dabei findet eine starke Oberflächenvergrößerung der Lösung 12 und damit eine Vergrößerung der Grenzfläche 16 zum Gas 18 und dadurch eine effektive Oxidation des Katalysators statt. Mittels des Wärmetauschers 32 wird die Lösung 12 auf einer konstanten Temperatur gehalten.

Nach Durchlaufen des statischen Mischers 34 wird die Lösung 12 mit dem darin in Bläschen verteilten Gas 18 durch die Flüssigkeitsleitung 28 in das Gefäß 10 eingeleitet und in die Lösung 12 eingestrahlt. Dies bewirkt neben der im statischen Mischer 34 erfolgenden intensiven Durchmischung eine weitere intensive Durchmischung der Lösung 12 mit dem Gas 18, so dass dadurch auch in dem Gefäß 10 eine Oxidation des Katalysators erfolgt. Das unter dem Druck stehende Gas 18 über der Lösung 12 wird über die Abführleitung 20 dem Separator 22 zugeführt, in welchem $CO_2$ und/oder CO mittels einer Membran oder Druckwechsel-Adsorption von dem Gas 18 abgesondert wird. Das Gas 18 wird dann über einen Kompressor 26 und die Einspeiseleitung 23 in die Zuführleitung 14 und von dort in den statischen Mischer 34 eingespeist. Der Kompressor 26 fördert das unter Druck stehende Gas 18 im so gebildeten Kreislauf. Der nicht für die Oxidation des Katalysators benötigte Sauerstoff in dem Gas 18 wird auf diese Weise im Kreis geführt, während das durch die Reaktion gebildete CO und/oder $CO_2$ im Separator 22 abgesondert wird. Dadurch wird vermieden, dass der Volumenanteil von CO und $CO_2$ zusammen an dem Gas 55 % übersteigt.

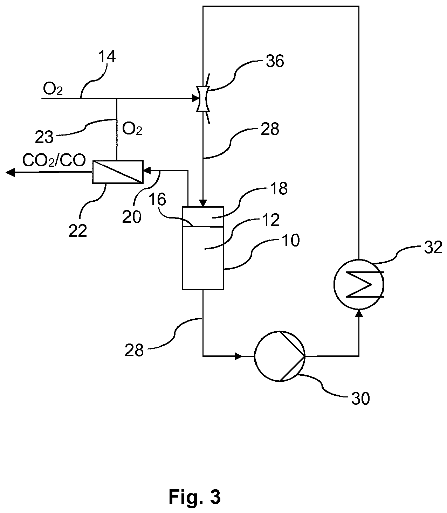

Die in Fig. 3 dargestellte Vorrichtung unterscheidet sich von der in Fig. 2 dargestellten Vorrichtung dadurch, dass statt des statischen Mischers 34 eine Venturi-Düse 36 vorgesehen ist. Die über die Flüssigkeitsleitung 28 der Venturi-Düse 36 zugeführte Lösung 12 durchströmt die Venturi-Düse 36 und reißt dabei das über die Zuführleitung 14 zugeführte Gas 18 durch den Venturi-Effekt mit. Die Venturi-Düse 36 bewirkt eine intensive Durchmischung des Gases 18 mit der Lösung 12. Eine weitere intensive Durchmischung mit der Lösung 12 wird durch das Einstrahlen der Lösung 12 mit dem darin in Bläschen verteilten Gas 18 aus der Venturi-Düse 36 in die Lösung 12 im Gefäß 10 bewirkt. Das Einstrahlen der Lösung 12 kann über die Flüssigkeitsleitung 28 erfolgen. Es ist jedoch auch möglich, dass die Venturi-Düse 36 direkt am Gefäß 10 angeordnet ist und das Einstrahlen der Lösung 12 direkt aus der Venturi-Düse 36 in die Lösung 12 erfolgt.

Da die Venturi-Düse 36 eine Förderung des Gases 18 in einem Kreislauf bewirkt, kann hier ein Kompressor 26 entfallen. Durch das Vorsehen der Venturi-Düse 36 wird die Vorrichtung vereinfacht und dadurch kostengünstiger. Es ist auch möglich, die Lösung 12 zunächst durch einen statischen Mischer 34 und dann durch eine Venturi-Düse 36 oder zunächst durch eine Venturi-Düse 36 und dann durch einen statischen Mischer 34 zu leiten und das Gas 18 durch eine aufgeteilte Zuführleitung 14 sowohl dem statischen Mischer 34 als auch der Venturi-Düse 36 zuzuführen. Auch in diesem Fall kann ggf. ein Kompressor 26 entfallen.

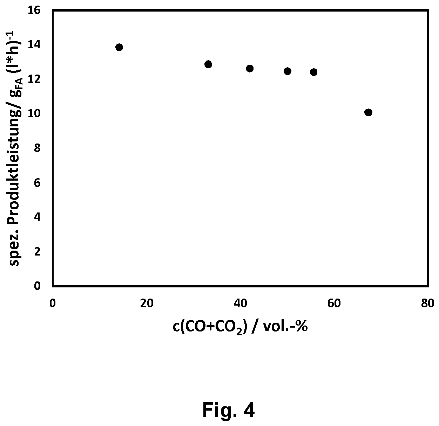

Fig. 4 zeigt das Ergebnis der Bestimmung der Ausbeute an Ameisensäure bei Zucker als bei dem erfindungsgemäßen Verfahren eingesetzten Edukt bei verschiedenen definierten Volumenanteilen von CO und $CO_2$ zusammen

an dem Gas. Die Ausbeute ist dabei als spezifischen Produktleistung (Raum-Zeit-Ausbeute) angegeben. Dabei zeigt sich, dass bei diesem Substrat die Ausbeute an Ameisensäure ab einem Volumenanteil von CO und $CO_2$ zusammen von 55 % deutlich abfällt. Je nach Substrat kann dieser Abfall auch bei einem niedrigeren oder höheren Volumenanteil erfolgen. Der höchste Volumenanteil, bei dem der Abfall erfolgt, liegt bei 80 %.

Oxidation eines Gärrests

Zu 2.800 g einer 21 g $H_8PMo_7V_5O_{40}$ und 50,6 g $H_2SO_4$ enthaltenden wässrigen Lösung 12, werden 195 g separierter Gärrest (Trockensubstanz) aus einer Biogasanlage hinzugegeben. In einem Rührkessel-Autoklaven wird die Lösung 12 mit Sauerstoff unter einem Sauerstoffpartialdruck von 4 bar in Kontakt gebracht und bei 130 °C gerührt. Zum Zuführen von $O_2$ und Abführen von entstandenem $CO_2$ wird mit einem Gasstrom von 0,7 ln $O_2$ pro Minute gespült. Nach nur 5,5 Stunden Reaktionszeit entspricht der Ameisensäuregehalt in der Reaktionslösung bereits einer Ausbeute von ca. 24 %.

Oxidation von Getreide-Schlempe

Die Reaktion wird wie bei der Oxidation des Gärrests durchgeführt. Abweichend davon werden 98 g Toluol-sulfonsäure statt $H_2SO_4$ sowie 195 g Trockenschlempe aus einer Bio-Ethanolherstellung statt des Gärrests eingesetzt. Weiterhin abweichend wird die Lösung 12 mit Sauerstoff unter einem Sauerstoffpartialdruck von 8 bar in Kontakt gebracht. Nach nur 2 Stunden Reaktionszeit entspricht der Ameisensäuregehalt in der Reaktionslösung bereits einer Ausbeute von ca. 51 %.

Oxidation von Rindergülle

Die Reaktion wird wie bei der Oxidation des Gärrests durchgeführt. Abweichend davon werden 195 g Rindergülle (Trockensubstanz) statt des Gärrests eingesetzt. Weiterhin abweichend wird die Temperatur nach 1 Stunde von 110 °C auf 120 °C und nach weiteren 2 Stunden auf 130 °C erhöht. Nach 4 Stunden Reaktionszeit entspricht der Ameisensäuregehalt in der Reaktionslösung einer Ausbeute von ca. 23 %.

Oxidation von Bananenschalen

Zu 2.400 g einer 43,7 g $H_8PMo_7V_5O_{40}$ und 82,7 g Toluolsulfonsäure enthaltenden wässrigen Lösung 12, werden 90 g getrockneter Bananenschale hinzugegeben. In einem Rührkessel-Autoklaven wird die Lösung 12 mit Sauerstoff unter einem Sauerstoffpartialdruck von 10 bar in Kontakt gebracht und bei 90 °C gerührt. Zum Zuführen von $O_2$ und abführen von entstandenem $CO_2$ wird mit einem Gasstrom von 1,0 ln $O_2$ pro Minute gespült. Nach 5 Stunden Reaktionszeit im Batch-Betrieb entspricht der Ameisensäuregehalt in der Lösung 12 einer Ausbeute von ca. 36 %.

Oxidation von Obsttrester

Die Reaktion wird wie bei der Oxidation der Bananenschalen durchgeführt. Abweichend davon werden 90 g getrockneten Tresters statt der Bananenschalen eingesetzt. Nach 5 Stunden Reaktionszeit im Batch-Betrieb entspricht der Ameisensäuregehalt in der Lösung 12 einer Ausbeute von ca. 25 %.

Oxidation von Saccharose ohne Gasaustausch

Die Reaktion wird wie die Oxidation des Gärrests durchgeführt. Abweichend hiervon werden 1.652 g Wasser, 3 g $H_8PMo_7V_5O_{40}$, 5,7 g Toluolsulfonsäure und 200 g Saccharose eingesetzt. Weiterhin abweichend wird die Lösung 12 mit Sauerstoff unter einem Sauerstoffpartialdruck von 10 bar in Kontakt gebracht, ohne dass während der Reaktion ein Gasaustausch erfolgt und bei 90 °C gerührt. Nach 4 Stunden Reaktionszeit entspricht der Ameisensäuregehalt in der Reaktionslösung einer Ausbeute von ca. 30 %.

Oxidation von Saccharose mit permanentem $O_2$ Strom

Die Reaktion wird wie die Oxidation des Gärrests durchgeführt. Abweichend hiervon werden 1.652 g Wasser, 3 g $H_8PMo_7V_5O_{40}$, 5,7 g Toluolsulfonsäure und 200 g Saccharose eingesetzt. Weiterhin abweichend wird die Lösung 12 mit Sauerstoff und einem Sauerstoffpartialdruck von 10 bar in Kontakt gebracht und bei 90 °C gerührt. Zum Zuführen von $O_2$ und Abführen von entstandenem $CO_2$ wird mit einem Gasstrom von 5 ln $O_2$ pro Minute gespült. Nach 4 Stunden Reaktionszeit entspricht der Ameisensäuregehalt in der Reaktionslösung einer Ausbeute von ca. 40 %.

Bezugszeichenliste

| | |
|---|---|
| 10 | Gefäß |
| 12 | Lösung |
| 14 | Zuführleitung |
| 16 | Grenzfläche |
| 18 | Gas |
| 20 | Abführleitung |
| 22 | Separator |
| 23 | Einspeiseleitung |
| 24 | Rückführleitung |
| 26 | Kompressor |
| 28 | Flüssigkeitsleitung |
| 30 | Pumpe |
| 32 | Wärmetauscher |
| 34 | statischer Mischer |
| 36 | Venturi-Düse |

**Patentansprüche**

1. Verfahren zur katalytischen Erzeugung von Ameisensäure und Regeneration des dabei eingesetzten Katalysators, wobei ein als Katalysator dienendes Vanadyl-Ion, Vanadat-Ion oder Polyoxometallat-Ion der allgemeinen Formel $[PMo_xV_yO_{40}]^{n-}$ bei einer Temperatur oberhalb von 70 °C und unterhalb von 160 °C mit einem alpha-Hydroxyaldehyd, einer alpha-Hydroxycarbonsäure, einem Kohlenhydrat, einem Glycosid oder einem eine Kohlenstoffkette enthaltenden Polymer mit mindestens einer wiederholt als Substituent an die Kohlenstoffkette gebundenen OH-Gruppe und/oder mit einem wiederholt in der Kohlenstoffkette enthaltenen O-, N- oder S-Atom in einer flüssigen Lösung (12) in einem Gefäß (10) in Kontakt gebracht wird, wobei $6 \leq x \leq 11$ und $1 \leq y \leq 6$ und $3 < n < 10$ und $x + y = 12$, wobei n, x und y jeweils eine ganze Zahl ist, wobei der dabei reduzierte Katalysator durch Oxidation wieder in seinen Ausgangszustand versetzt wird, **dadurch gekennzeichnet, dass** die Lösung (12) dazu mit einem einen Volumenanteil von mindestens 18 % Sauerstoff enthaltenden Gas (18) bei einem Druck von mindestens 2 bar und höchstens 16 bar mittels einer Mischvorrichtung oder über eine flüssigkeitsundurchlässige gasdurchlässige Membran in Kontakt gebracht wird, wobei bei der Reaktion entstehendes und in das Gas (18) übergehendes CO und/oder $CO_2$ in einer solchen Menge abgeführt wird, dass ein Volumenanteil von CO und $CO_2$ zusammen an dem Gas (18) 80 % nicht übersteigt.

2. Verfahren nach Anspruch 1, **dadurch gekennzeichnet, dass** der Volumenanteil von CO und $CO_2$ zusammen an dem Gas (18) mindestens 20 % beträgt und/oder dass das bei der Reaktion entstehende und in das Gas (18) übergehende CO und/oder $CO_2$ in einer solchen Menge abgeführt wird, dass der Volumenanteil von CO und $CO_2$ zusammen an dem Gas (18) 70 %, 60 %, 55 %, 50 %, 45 %, 40 %, 35 %, 30 %, 25 % oder 20 % nicht übersteigt.

3. Verfahren nach einem der vorhergehenden Ansprüche, **dadurch gekennzeichnet, dass** das bei der Reaktion entstehende und in das Gas (18) übergehendes CO und/oder $CO_2$ dadurch in einer solchen Menge abgeführt wird, dass der Volumenanteil von CO und $CO_2$ zusammen an dem Gas (18) 80 %, 70 %, 60 %, 55 %, 50 %, 45 %, 40 %, 35 %, 30 %, 25 % oder 20 % nicht übersteigt, dass das die Lösung kontaktierende Gas (18) oder zumindest ein Teil dieses Gases (18) permanent oder stoßweise spätestens bei Erreichen des Volumenanteils von 80 %, 70 %, 60 %, 55 %, 50 %, 45 %, 40 %, 35 %, 30 %, 25 % oder 20 % durch frisches Gas (18) ersetzt wird oder dass das CO und/oder das $CO_2$ von dem Gas (18) abgesondert wird.

4. Verfahren nach einem der vorhergehenden Ansprüche, **dadurch gekennzeichnet, dass** das Vanadyl-Ion, Vanadat-Ion oder Polyoxometallat-Ion bei einer Temperatur oberhalb von 80 °C, insbesondere oberhalb von 90 °C, und/oder unterhalb von 150 °C, insbesondere unterhalb von 140 °C, mit dem alpha-Hydroxyaldehyd, der alpha-Hydroxycarbonsäure, dem Kohlenhydrat, dem Glycosid oder dem Polymer in Kontakt gebracht wird.

5. Verfahren nach einem der vorhergehenden Ansprüche, **dadurch gekennzeichnet, dass** der Druck mindestens 3 bar, mindestens 4 bar oder mindestens 5 bar und/oder höchstens 15 bar, 12 bar, 10 bar, 9 bar, 8 bar, 7 bar, oder 6 bar beträgt.

6. Verfahren nach einem der vorhergehenden Ansprüche, **dadurch gekennzeichnet, dass** das bei der Reaktion entstehende und in das Gas übergehende CO und/oder $CO_2$ in einer solchen Menge abgeführt wird, dass bei auf einen Höchstwert beschränktem Druck oder bei konstant gehaltenem Druck ein Sauerstoffpartialdruck in dem Gas durch das CO und/oder $CO_2$ um nicht mehr als 10 % vermindert wird.

7. Verfahren nach einem der vorhergehenden Ansprüche, **dadurch gekennzeichnet, dass** zur Oxidation des Katalysators ein Teil der flüssigen Lösung (12) aus dem Gefäß (10) ausgeleitet, mit dem Gas (18) in Kontakt gebracht und anschließend wieder dem Rest der flüssigen Lösung (12) zugeführt wird.

8. Verfahren nach einem der vorhergehenden Ansprüche, **dadurch gekennzeichnet, dass** in dem Gas entstandene dampfförmige Ameisensäure, insbesondere vor dem Ersetzen des die Lösung kontaktierenden Gases (18) durch frisches Gas (18), mittels eines zur Absorption von Ameisensäure geeigneten Absorptionsmittels, insbesondere einem linearen Alkohol, insbesondere 1-Pentanol, 1-Hexanol, 1-Heptanol, 1-Oktanol, oder 1-Dekanol, oder einem Amid, insbesondere einem N,N-Dialkylcarbonsäureamid, insbesondere Dipentylformamid, N-(n-Hexadecyl)formamid, N,N-Di-n-butylformamid (DBFA), N-Di-n-acetamid, N-Methyl-N-hetpylformamid, N-n-butyl-N-2-ethylhexylformamid oder N-n-butyl-N-cyclohexylformamid, aus dem Gas (18) absorbiert und anschließend davon desorbiert wird, oder aus dem Gas (18) von einer Base, insbesondere einer wässrigen Lösung von NaOH oder KOH, absorbiert und die daraus resultierende Salzlösung abgeleitet wird oder am Gefäß (10) oder außerhalb des Gefäßes (10) kondensiert wird, wobei ein dadurch entstehendes Kondensat in das Gefäß zurückgeleitet, zur Absonderung der Ameisensäure von dem darin enthaltenen Wasser einer Extraktion, insbesondere mittels eines der genannten Absorptionsmittel, zugeführt oder zur Absonderung der Ameisensäure abgeleitet wird.

9. Verfahren nach einem der vorhergehenden Ansprüche, **dadurch gekennzeichnet, dass** die Mischvorrichtung einen statischen Mischer (34), eine Reaktionsmischpumpe, eine Düse, insbesondere eine Venturi-Düse (36) oder eine Sprühdüse, und/oder einen Gaseintragsrührer umfasst.

10. Verfahren nach einem der vorhergehenden Ansprüche, wobei die Mischvorrichtung so ausgelegt ist und so betrieben wird oder die Membran so aufgebaut ist, dass dadurch die Oberfläche der Lösung mindestens um den Faktor 1000 vergrößert wird.

11. Verfahren nach einem der vorhergehenden Ansprüche, **dadurch gekennzeichnet, dass** das CO und/oder das $CO_2$ von dem Gas (18) mittels einer für das CO und/oder das $CO_2$ durchlässigen und für $O_2$ undurchlässigen oder nur eingeschränkt durchlässigen Membran, einer Kombination von zwei oder mehreren Membranen unterschiedlicher Durchlässigkeit oder mittels einer Druckwechsel-Adsorption abgesondert wird.

12. Verfahren nach einem der vorhergehenden Ansprüche, **dadurch gekennzeichnet, dass** das Gas (18), insbesondere ohne einen Druckabfall von mehr als 2,5 bar, in einem Kreislauf geführt wird.

13. Verfahren nach einem der vorhergehenden Ansprüche, **dadurch gekennzeichnet, dass** das Verfahren als kontinuierlicher Prozess durchgeführt wird.

14. Verfahren nach einem der vorhergehenden Ansprüche, **dadurch gekennzeichnet, dass** der Katalysator und die Ameisensäure mittels mindestens eines die Ameisensäure und den Katalysator extrahierenden polaren organischen Extraktionsmittels, welches beim Mischen mit der Lösung (12) eine Phasengrenze zwischen der Lösung (12) und dem Extraktionsmittel bildet, aus der Lösung (12) oder aus einer danach der Lösung (12) wieder zugeführten Teilmenge der Lösung (12), insbesondere unter Aufrechterhaltung eines pH-Werts von höchstens 3, insbesondere höchstens 2,5, in der Lösung (12), abgesondert werden, wobei das Extraktionsmittel ein solches ist, welches für eine Extraktion des in einer Konzentration von 1,5 Gew.-% in Wasser enthaltenen Katalysators bei 40 °C einen Verteilungskoeffizienten für den Katalysator aufweist, welcher mindestens um den Faktor 7 größer ist als ein Verteilungskoeffizient für eine Extraktion der in einer Konzentration von 5 Gew.-% in Wasser enthaltenen Ameisensäure bei 40 °C, und wobei das Extraktionsmittel vor der Extraktion mit dem Katalysator gesättigt wird oder wobei der abgesonderte Katalysator aus dem Extraktionsmittel abgesondert und der flüssigen Lösung (12) in dem Gefäß (10) zugeführt wird.

15. Verfahren nach Anspruch 14, **dadurch gekennzeichnet, dass** das Absondern in einem zwei-stufigen Prozess erfolgt, indem die Lösung (12) in einem ersten Schritt der Extraktion mit einer ersten Menge des Extraktionsmittels für eine erste Zeit zur Extraktion des Katalysators und in einem zweiten Schritt der Extraktion mit einer zweiten Menge des Extraktionsmittels für eine zweite Zeit zur Extraktion der Ameisensäure extrahiert wird, wobei der in dem ersten Schritt der Extraktion extrahierte Katalysator wieder der flüssigen Lösung (12) in dem Gefäß (10) zugeführt wird.

16. Verfahren nach Anspruch 14 oder 15, **dadurch gekennzeichnet, dass** dem Extraktionsmittel ein, insbesondere unpolares, Additiv zugesetzt wird.

17. Verfahren nach einem der Ansprüche 14 bis 16, **dadurch gekennzeichnet, dass** das Extraktionsmittel ein Amid, insbesondere ein N,N-Dialkylcarbonsäureamid, insbesondere Dipentylformamid, N-(n-Hexadecyl)formamid, N,N-Di-n-butylformamid (DBFA), N-Di-n-acetamid, N-Methyl-N-hetpylformamid, N-n-butyl-N-2-ethylhexylformamid oder N-n-butyl-N-cyclohexylformamid ist.

18. Verfahren nach einem der Ansprüche 14 bis 17, **dadurch gekennzeichnet, dass** der Katalysator mittels Fällung als Salz, insbesondere gleichzeitig mit einer Fällung von extrahierter Ameisensäure als Formiat, oder mittels einer weiteren Extraktion mit einem polaren weiteren Extraktionsmittel, insbesondere einem Lösungsmittel der Lösung (12), und einem Temperaturwechsel des Extraktionsmittels und/oder einer Erhöhung des pH-Werts des Extraktionsmittels, insbesondere durch Zusatz eines Carbonats und/oder eines Hydroxids, abgesondert wird.

## Claims

1. A method for catalytically producing formic acid and regenerating the catalyst employed, where a vanadyl ion, vanadate ion or polyoxometalate ion of the general formula $[PMo_xV_yO_{40}]^{n-}$ serving as catalyst is contacted at a temperature above 70°C and below 160°C with an alpha-hydroxyaldehyde, an alpha-hydroxycarboxylic acid, a carbohydrate, a glycoside or a polymer containing a carbon chain and having at least one OH group bonded repeatedly as substituent to the carbon chain and/or having an O, N or S atom present repeatedly in the carbon chain, in a liquid solution (12) in a vessel (10), where $6 \leq x \leq 11$ and $1 \leq y \leq 6$ and $3 < n < 10$ and $x + y = 12$, where n, x and y are each an integer, where the catalyst reduced is returned by oxidation to its original state, **characterized in that** the solution (12) for this purpose is contacted with a gas (18) comprising a volume fraction of at least 18% of oxygen at a pressure of at least 2 bar and at most 16 bar, by means of a mixing apparatus or via a liquid-impermeable, gas-permeable membrane, where CO and/or $CO_2$ formed in the reaction and passing into the gas (18) are/is taken off in a quantity such that the volume fraction of CO and $CO_2$ together in the gas (18) does not exceed 80%.

2. The method as claimed in claim 1, **characterized in that** the volume fraction of CO and $CO_2$ together in the gas (18) is at least 20% and/or **in that** the CO and/or $CO_2$ formed in the reaction and passing into the gas (18) are/is taken off in a quantity such that the volume fraction of CO and $CO_2$ together in the gas (18) does not exceed 70%, 60%, 55%, 50%, 45%, 40%, 35%, 30%, 25% or 20%.

3. The method as claimed in either of the preceding claims, **characterized in that** the CO and/or $CO_2$ formed in the reaction and passing into the gas (18) are/is taken off in a quantity such that the volume fraction of CO and $CO_2$ together in the gas (18) does not exceed 80%, 70%, 60%, 55%, 50%, 45%, 40%, 35%, 30%, 25% or 20% by using fresh gas (18) to replace the gas (18) contacting the solution, or at least a part of this gas (18), permanently or intermittently, no later than on attainment of the volume fraction of 80%, 70%, 60%, 55%, 50%, 45%, 40%, 35%, 30%, 25% or 20%, or by separating the CO and/or the $CO_2$ from the gas (18).

4. The method as claimed in any of the preceding claims, **characterized in that** the vanadyl ion, vanadate ion or polyoxometalate ion is contacted at a temperature above 80°C, more particularly above 90°C, and/or below 150°C, more particularly below 140°C, with the alpha-hydroxyaldehyde, the alpha-hydroxycarboxylic acid, the carbohydrate, the glycoside or the polymer.

5. The method as claimed in any of the preceding claims, **characterized in that** the pressure is at least 3 bar, at least 4 bar or at least 5 bar and/or at most 15 bar, 12 bar, 10 bar, 9 bar, 8 bar, 7 bar or 6 bar.

6. The method as claimed in any of the preceding claims, **characterized in that** the CO and/or $CO_2$ formed in the reaction and passing into the gas are/is taken off in a quantity such that, with a pressure restricted to a maximum value or with pressure held constant, the oxygen partial pressure in the gas is diminished by the CO and/or $CO_2$ by

not more than 10%.

7. The method as claimed in any of the preceding claims, **characterized in that** for the oxidation of the catalyst, a portion of the liquid solution (12) is led out of the vessel (10), contacted with the gas (18), and subsequently supplied again to the remainder of the liquid solution (12).

8. The method as claimed in any of the preceding claims, **characterized in that** vaporous formic acid formed in the gas, more particularly before the replacement of the solution-contacting gas (18) by fresh gas (18), is absorbed from the gas (18) by means of an absorbent suitable for absorbing formic acid, more particularly a linear alcohol, more particularly 1-pentanol, 1-hexanol, 1-heptanol, 1-octanol or 1-decanol, or an amide, more particularly an N,N-dialkylcarboxamide, more particularly dipentylformamide, N-(n-hexadecyl)formamide, N,N-di-n-butylformamide (DBFA), N-di-n-acetamide, N-methyl-N-heptylformamide, N-n-butyl-N-2-ethylhexyl-formamide or N-n-butyl-N-cyclohexylformamide, and is subsequently desorbed therefrom, or is absorbed from the gas (18) by a base, more particularly an aqueous solution of NaOH or KOH, and the resulting salt solution is led off or is condensed at the vessel (10) or outside the vessel (10), where a condensate formed as a result is led back into the vessel, supplied to an extraction, more particularly by means of one of the stated absorbents, to separate the formic acid from the water contained therein, or led off for the separation of the formic acid.

9. The method as claimed in any of the preceding claims, **characterized in that** the mixing apparatus comprises a static mixer (34), a reactive mixing pump, a nozzle, more particularly a Venturi nozzle (36) or a spraying nozzle, and/or a gas introduction stirrer.

10. The method as claimed in any of the preceding claims, where the mixing apparatus is designed in such a way and operated in such a way, or the membrane is constructed in such a way, that the surface area of the solution is increased by a factor of at least 1000 as a result.

11. The method as claimed in any of the preceding claims, **characterized in that** the CO and/or the $CO_2$ are/is separated from the gas (18) by means of a membrane which is permeable for the CO and/or the $CO_2$ and impermeable or of only limited permeability for $O_2$, a combination of two or more membranes with different permeabilities, or by means of a pressure swing adsorption.

12. The method as claimed in any of the preceding claims, **characterized in that** the gas (18) is guided in a circuit, in particular without a pressure drop of more than 2.5 bar.

13. The method as claimed in any of the preceding claims, **characterized in that** the method is carried out as a continuous process.

14. The method as claimed in any of the preceding claims, **characterized in that** the catalyst and the formic acid are separated from the solution (12) or from a portion of the solution (12) that is subsequently resupplied to the solution (12), by means of at least one polar organic extractant which extracts the formic acid and the catalyst and which, on mixing with the solution (12), forms a phase boundary between the solution (12) and the extractant, more particularly with the pH being maintained at not more than 3, more particularly not more than 2.5, in the solution (12), where the extractant is one which, for extraction of the catalyst present at a concentration of 1.5 wt% in water, has a partition coefficient for the catalyst at 40°C that is greater by a factor of at least 7 than a partition coefficient for extraction of the formic acid present at a concentration of 5 wt% in water at 40°C, and where the extractant before the extraction is saturated with the catalyst or where the separated catalyst is separated from the extractant and resupplied to the liquid solution (12) in the vessel (10).

15. The method as claimed in claim 14, **characterized in that** the separation takes place in a two-stage process, by extracting the solution (12) in a first extraction step with a first quantity of the extractant for a first time, to extract the catalyst, and extracting the solution in a second extraction step with a second quantity of the extractant for a second time, to extract the formic acid, where the catalyst extracted in the first extraction step is supplied again to the liquid solution (12) in the vessel (10).

16. The method as claimed in claim 14 or 15, **characterized in that** the extractant is admixed with an additive, more particularly an apolar additive.

17. The method as claimed in any of claims 14 to 16, **characterized in that** the extractant is an amide, more particularly an N,N-dialkylcarboxamide, more particularly dipentylformamide, N-(n-hexadecyl)formamide, N,N-di-n-butylformamide (DBFA), N-di-n-acetamide, N-methyl-N-heptylformamide, N-n-butyl-N-2-ethylhexyl-formamide or N-n-butyl-N-cyclohexylformamide.

18. The method as claimed in any of claims 14 to 17, **characterized in that** the catalyst is separated by means of precipitation as a salt, more particularly simultaneously with a precipitation of extracted formic acid as formate, or by means of further extraction with a polar further extractant, more particularly a solvent of the solution (12), and with a temperature change of the extractant and/or an increase in the pH of the extractant, more particularly through addition of a carbonate and/or a hydroxide.

## Revendications

1. Procédé de génération catalytique d'acide formique et de régénération du catalyseur utilisé ce faisant, dans lequel un ion vanadyle, ion vanadate ou ion polyoxométallate servant de catalyseur de la formule générale $[PMo_xV_yO_{40}]^{n-}$ est amené en contact à une température au-dessus de 70°C et en dessous de 160°C avec un alpha-hydroxyaldéhyde, un acide alpha-hydroxycarboxylique, un hydrate de carbone, un glycoside ou un polymère contenant une chaîne de carbones avec au moins un groupe OH fixé de manière répétée en tant que substituant à la chaîne de carbones et/ou avec un atome de O, N ou S contenu de manière répétée dans la chaîne de carbones dans une solution liquide (12) dans un récipient (10), dans lequel $6 \leq x \leq 11$ et $1 \leq y \leq 6$ et $3 < n < 10$ et $x + y = 12$, dans lequel n, x et y sont chacun un nombre entier, dans lequel le catalyseur réduit ce faisant est remis dans son état de départ par oxydation, **caractérisé en ce que** la solution (12) est amenée en contact à cet effet avec un gaz (18) contenant une proportion volumique d'au moins 18 % d'oxygène à une pression d'au moins 2 bars et d'au plus 16 bars au moyen d'un dispositif de mélange ou par le biais d'une membrane imperméable au liquide, perméable au gaz, dans lequel du CO et/ou $CO_2$ apparaissant lors de la réaction et passant dans le gaz (18) est évacué en une quantité telle qu'une proportion volumique de CO et $CO_2$ ensemble dans le gaz (18) ne dépasse pas 80 %.

2. Procédé selon la revendication 1, **caractérisé en ce que** la proportion volumique de CO et $CO_2$ ensemble dans le gaz (18) se monte à au moins 20 % et/ou que du CO et/ou $CO_2$ apparaissant lors de la réaction et passant dans le gaz (18) est évacué en une quantité telle que la proportion volumique de CO et $CO_2$ ensemble dans le gaz (18) ne dépasse pas 70 %, 60 %, 55 %, 50 %, 45 %, 40 %, 35 %, 30 %, 25 % ou 20 %.

3. Procédé selon une des revendications précédentes, **caractérisé en ce que** du CO et/ou $CO_2$ apparaissant lors de la réaction et passant dans le gaz (18) est évacué en une quantité telle que la proportion volumique de CO et $CO_2$ ensemble dans le gaz (18) ne dépasse pas 80 %, 70 %, 60 %, 55 %, 50 %, 45 %, 40 %, 35 %, 30 %, 25 % ou 20 %, que le gaz (18) se trouvant en contact avec la solution ou au moins une partie de ce gaz (18) est remplacé en permanence ou par à-coup au plus tard lors de l'atteinte de la proportion volumique de 80 %, 70 %, 60 %, 55 %, 50 %, 45 %, 40 %, 35 %, 30 %, 25 % ou 20 % par du gaz frais (18) ou que le CO et/ou le $CO_2$ est séparé du gaz (18).

4. Procédé selon une des revendications précédentes, **caractérisé en ce que** l'ion vanadyle, l'ion vanadate ou l'ion polyoxométallate est amené en contact à une température au-dessus de 80°C, notamment au-dessus de 90°C, et/ou en dessous de 150°C, notamment en dessous de 140°C, avec l'alpha-hydroxyaldéhyde, l'acide alpha-hydroxycarboxylique, l'hydrate de carbone, le glycoside ou le polymère.

5. Procédé selon une des revendications précédentes, **caractérisé en ce que** la pression se monte à au moins 3 bars, au moins 4 bars ou au moins 5 bars et/ou au plus 15 bars, 12 bars, 10 bars, 9 bars, 8 bars, 7 bars ou 6 bars.

6. Procédé selon une des revendications précédentes, **caractérisé en ce que** du CO et/ou $CO_2$ apparaissant lors de la réaction et passant dans le gaz est évacué en une quantité telle qu'une pression partielle d'oxygène dans le gaz est réduite de pas plus de 10 % par le CO et/ou $CO_2$ à une pression limitée à une valeur maximale ou à une pression maintenue constante.

7. Procédé selon une des revendications précédentes, **caractérisé en ce qu'**une partie de la solution liquide (12) est déviée du récipient (10) en vue de l'oxydation du catalyseur, amenée en contact avec le gaz (18) et ensuite ramenée au reste de la solution liquide (12).

8. Procédé selon une des revendications précédentes, **caractérisé en ce que** de l'acide formique sous forme de vapeur apparu dans le gaz, notamment avant le remplacement du gaz (18) se trouvant en contact avec la solution par du gaz frais (18), est absorbé du gaz (18) au moyen d'un agent d'absorption approprié pour l'absorption d'acide formique, notamment d'un alcool linéaire, notamment de 1-pentanol, 1-hexanol, 1-heptanol, 1-octanol ou 1-décanol, ou d'un amide, notamment d'un amide d'acide N,N-dialkylcarboxylique, notamment de dipentylformamide, N-(n-hexadécyl)formamide, N,N-di-n-butylformamide (DBFA), N-di-n-acétamide, N-méthyl-N-heptylformamide, N-n-butyl-N-2-éthylhexylformamide ou N-n-butyl-N-cyclohexylformamide, puis désorbé de celui-ci, ou est absorbé du gaz (18) par une base, notamment une solution aqueuse de NaOH ou KOH et la solution de sel résultant de celle-ci est évacuée ou est condensée au niveau du récipient (10) ou en dehors du récipient (10), dans lequel un condensat apparaissant ce faisant est renvoyé dans le récipient, amené en vue de la séparation de l'acide formique de l'eau contenue dans celui-ci à une extraction, notamment au moyen d'un des agents d'absorption cités, ou évacué en vue de la séparation de l'acide formique.

9. Procédé selon une des revendications précédentes, **caractérisé en ce que** le dispositif de mélange comprend un mélangeur statique (34), une pompe de mélange de réaction, une buse, notamment une buse Venturi (36) ou une buse de pulvérisation, et/ou un agitateur à alimentation gazeuse.

10. Procédé selon une des revendications précédentes, **caractérisé en ce que** le dispositif de mélange est conçu et est exploité ou la membrane est construite de sorte que la surface de la solution est ce faisant agrandie au moins du facteur 1000.

11. Procédé selon une des revendications précédentes, **caractérisé en ce que** le CO et/ou le $CO_2$ est séparé du gaz (18) au moyen d'une membrane perméable pour le CO et/ou le $CO_2$ et imperméable ou perméable de manière seulement limitée pour du $O_2$, d'une combinaison de deux ou plusieurs membranes de perméabilité différente ou au moyen d'une adsorption à changement de pression.

12. Procédé selon une des revendications précédentes, **caractérisé en ce que** le gaz (18) est guidé dans un circuit, notamment sans une chute de pression de plus de 2,5 bars.

13. Procédé selon une des revendications précédentes, **caractérisé en ce que** le procédé est réalisé en tant que processus continu.

14. Procédé selon une des revendications précédentes, **caractérisé en ce que** le catalyseur et l'acide formique sont séparés de la solution (12) ou d'une quantité partielle de la solution (12) ramenée ensuite à la solution (12) au moyen d'au moins un agent d'extraction organique polaire extrayant l'acide formique et le catalyseur qui forme lors du mélange avec la solution (12) une limite de phase entre la solution (12) et l'agent d'extraction, notamment en conservant une valeur de pH d'au plus 3, notamment d'au plus 2,5, dans la solution (12), dans lequel l'agent d'extraction est un agent qui présente pour une extraction du catalyseur contenu à une concentration 1,5 % en poids dans de l'eau à 40°C un coefficient de distribution pour le catalyseur qui est supérieur au moins du facteur 7 à un coefficient de distribution pour une extraction de l'acide formique contenu à une concentration de 5 % en poids dans de l'eau à 40°C, et dans lequel l'agent d'extraction est saturé avec le catalyseur avant l'extraction ou dans lequel le catalyseur séparé est séparé de l'agent d'extraction et amené à la solution liquide (12) dans le récipient (10).

15. Procédé selon la revendication 14, **caractérisé en ce que** la séparation s'effectue dans un processus à deux étapes **en ce que** la solution (12) est extraite dans une première étape de l'extraction avec une première quantité de l'agent d'extraction pour une première durée en vue de l'extraction du catalyseur et dans une seconde étape de l'extraction avec une seconde quantité de l'agent d'extraction pour une seconde durée en vue de l'extraction de l'acide formique, dans lequel le catalyseur extrait dans la première étape de l'extraction est ramené à la solution liquide (12) dans le récipient (10).

16. Procédé selon la revendication 14 ou 15, **caractérisé en ce qu'**un additif, notamment non polaire, est ajouté à l'agent d'extraction.

17. Procédé selon une des revendications 14 à 16, **caractérisé en ce que** l'agent d'extraction est un amide, notamment un amide d'acide N,N-dialkylcarboxylique, notamment dipentylformamide, N-(n-hexadécyl)formamide, N,N-di-n-butylformamide (DBFA), N-di-n-acétamide, N-méthyl-N-heptylformamide, N-n-butyl-N-2-éthylhexylformamide ou N-n-butyl-N-cyclohexylformamide.

18. Procédé selon une des revendications 14 à 17, **caractérisé en ce que** le catalyseur est séparé au moyen d'une précipitation en tant que sel, notamment simultanément avec une précipitation d'acide formique extrait en tant que formiate, ou au moyen d'une autre extraction avec un autre agent d'extraction polaire, notamment un solvant de la solution (12), et un changement de température de l'agent d'extraction et/ou une augmentation de la valeur de pH de l'agent d'extraction, notamment par ajout d'un carbonate et/ou d'un hydroxyde.

CO₂/CO

O₂

22

18

16

20

24

26

10

12

O₂

14

**Fig. 1**

14

O₂

23

26

34

28

CO₂/CO

O₂

20

22

16

18

12

32

28

10

30

**Fig. 2**

**Fig. 3**

**Fig. 4**

**EP 3 484 846 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2473467 B1
- DE 102011077232 A1
- DE 102014212995 A1
- WO 2016078698 A1